Europäisches Patentamt

European Patent Office

Office européen des brevets

(11) Publication number: **0 344 006**
**A2**

# EUROPEAN PATENT APPLICATION

(21) Application number: **89305349.6**

(22) Date of filing: **26.05.89**

(51) Int. Cl.⁴: **C 07 K 7/10**
**A 61 K 37/02**

(30) Priority: **27.05.88 US 199973**

(43) Date of publication of application:
**29.11.89 Bulletin 89/48**

(84) Designated Contracting States:
**AT BE CH DE ES FR GB GR IT LI LU NL SE**

(71) Applicant: **ORTHO PHARMACEUTICAL CORPORATION**
**Route 202**
**Raritan, NJ 08869-0602 (US)**

**CALIFORNIA INSTITUTE OF TECHNOLOGY**
**1201 East California Boulevard**
**Pasadena California 91125 (US)**

(72) Inventor: **Rao, Patricia E.**
**11 Penn-Lyle Road**
**Princeton Junction N.J. 08850 (US)**

**Jameson, Bradford A.**
**112 N. Michigan Avenue No. 14**
**Pasadena CA 91106 (US)**

**Kent, Stephen B.H.**
**615 W. California Boulevard**
**Pasadena CA 91105 (US)**

(74) Representative: **Fisher, Adrian John et al**
**CARPMAELS & RANSFORD 43 Bloomsbury Square**
**London WC1A 2RA (GB)**

(54) Peptides that block the binding of hiv-1 to th CD4 receptor protein.

(57) This invention concerns peptides corresponding to amino acid sequences of the CD4 receptor protein on T4 cells which are capable of binding the gp120 envelope protein of HIV-1. These peptides are capable of blocking the binding of HIV-1 to the CD4 receptor and are therefore useful in treating HIV-1 infection.

EP 0 344 006 A2

Description

## PEPTIDES THAT BLOCK THE BINDING OF HIV-1 TO THE CD4 RECEPTOR PROTEIN

Background of the Invention

This invention relates to novel peptides that are capable of blocking the binding of the human immunodeficiency virus (HIV) to the CD4 receptor of a T4 cell. The present invention also concerns methods of using these peptides to treat patients infected with HIV, as well as antibodies specific for the peptides, vaccines containing the antibodies and diagnostic kits containing the peptides.

HIV is the etiological agent of what is commonly known as Acquired Immunodeficiency Syndrome (AIDS). HIV is an RNA retrovirus that has been given different names over the past several years, such as human T-lymphotropic virus III (HTLV-III); lymphadenopathy-associated virus (LAV); or AIDS-associated retrovirus (ARV). At the present time, HIV is being referred to as HIV-1 in order to differentiate it from a related virus designated HIV-2 (or LAV-2). For the purposes of this disclosure, the presently accepted designation HIV-1 will be used to designate the viral agent that causes AIDS.

Infection with HIV-1 results in a serious immunosuppression in the infected patient which is caused by the depletion of helper/inducer T-lymphocytes that express the receptor for the virus. This receptor is commonly referred to as the CD4 (or T4) receptor protein. As the primary function of the immune system is to protect the body against invasive microbes, such immunosuppression results in a defect in the host defense mechanism that renders the host highly susceptible to "opportunistic" infections and neoplasms. Infections by HIV-1 have been fatal in the majority of reported cases. Therefore, much effort has been expended by the medical community in the search for an agent that can be used to halt or prevent the spread of HIV-1 infection. For a current review of HIV-1, see the following articles: Anthony S. Fauci, "The Human Immunodeficiency Virus: Infectivity and Mechanisms of Pathogenesis", Science 239:617-622 (February 5, 1988); and Robert C. Gallo, "The AIDS Virus", Scientific American, pp. 47-56 (January 1987).

As mentioned above, the primary cause of the immunosuppression resulting from HIV-1 infection is the depletion of the helper/inducer subset of T-lymphocytes, which express the CD4 phenotypic marker (the T4 cell). The T4 lymphocyte is a critical cell involved directly or indirectly in the induction of a wide array of immunologic functions of the immune system. Therefore, a functional defect of T4 cells results in a decrease in many different immune responses in an HIV-1 infected patient. The CD4 molecule is one of several non-polymorphic T-lymphocyte surface proteins that have been implicated in the mediation of efficient T cell-target cell interactions. Mature T-lymphocytes segregate into one of two classes: those that express the surface glycoprotein T4 and those that express the glycoprotein T8. The T4 molecules are predominantly expressed on helper T-lymphocytes, whereas T8 is expressed on cytotoxic and suppressor T cells. T4+ T-lymphocytes interact with target cells that express the major histocompatibility complex (MHC) class II gene products, whereas T8+ T-cells interact with targets expressing class I MHC molecules. The interactions of helper T-lymphocytes are largely restricted to antigen-bearing target cells expressing class II MHC proteins, whereas cytotoxic and suppressor T-cells are restricted to targets bearing class I MHC molecules. Thus, the CD4 molecule may serve as a general receptor recognizing molecules on the surface of target cells, as well as a receptor for the HIV-1.

CD4 is a large surface glycoprotein with partial sequence homology to immunoglobulins, and this molecule has been isolated and characterized. The CD4 molecule has a molecular weight of about 55 kilodaltons and the processed molecule has a total of 435 amino acid residues. The CD4 molecule consists of a signal peptide, a 374-amino acid extracellular region containing 4 immunoglobin-like domains, a transmembrane domain and a charged intracellular region of 40 amino acid residues. See Maddon, P. J., "The Isolation and Nucleotide Sequence of a cDNA Encoding the T-cell Surface Protein T4: A New Member of the Immunoglobulin Gene Family", Cell 42:93-104 (August, 1985).

The ability of HIV-1 to selectively infect, replicate in and destroy T4 cells in part explains the loss of T4 helper/inducer function characteristic of AIDS. Several investigators suggested that the CD4 molecule is the cellular receptor for HIV-1 after they noticed that antibodies specific for CD4 could block HIV-1 infection and syncytia formation. Dalgleish, A. et al., "The CD4 (T4) Antigen Is An Essential Component for the Receptor for the AIDS Retrovirus," Nature 312:763-766 (1985); Klatzmann, D. et al., Selective Tropism of Lymphadenopathy Virus (LAV) for Helper-Inducer T-Lymphocytes," Science 225:59-63 (1984); and McDougal, J. et al., "Cellular Tropism of the Human Retrovirus HTLV-III/LAV. I. Role of T Cell Activation and Expression of The T4 Antigen," J. Immunol. 135:3151-3162 (1985). These observations were confirmed by the detection of CD4 binding to gp120, the major envelope glycoprotein of HIV-1 (McDougal, J. et al., "Binding of HTLV-III/LAV to T4+ T cells By a Complex of the 110K Viral Protein and the T4 Molecule," Science 231:382-385 (1986)) and the finding that non-permissive human cells are rendered susceptible to HIV-1 infection after the stable expression of a CD4 complimentary DNA (Maddon, P. et al., "The T4 Gene Encodes the AIDS Virus Receptor and Its Expressed In the Immune System and the Brain," Cell 47:333-348 (1986)).

An important mechanism of cell death in HIV-1 infection involving CD4-envelope protein interaction is cell fusion. The high level of HIV-1 envelope glycoprotein gene expression in infected T4 cells, as manifested by the budding of viral particles from the plasma membrane, results in cell fusion with neighboring uninfected T4 cells and leads to the formation of multinucleated giant cells known as syncytia that comprise both infected and uninfected cells. Depending on the virus isolate in question, cytolysis and death of the fused cells occurs usually within 48 hours.

Various investigators have reported that HIV-1 infection can be blocked by a "soluble" form of the CD4 molecule. See, for example, the following recent articles: Smith, D. H., et al., "Blocking of HIV-1 Infectivity by a Soluble, Secreted Form of the CD4 Antigen", Science 238:1704-1707 (December, 18, 1987); Deen, K. C. et al., "A Soluble Form of CD4 (T4) Protein Inhibits AIDS Virus Infection", Nature 331:82-84 (January 7, 1988); Fisher, R. A. et al., "HIV Infection is Blocked in vitro by Recombinant Soluble CD4", Nature 331:76-78 (January 7, 1988); Hussey, R. E., "A Soluble CD4 Protein Selectively Inhibits HIV Replication and Syncytium Formation", Nature 331:78-81 (January 7, 1988); and Traunecker, A. et al., "Soluble CD4 Molecules Neutralize Human Immunodeficiency Virus Type I", Nature 331:84-86 (January 7, 1988). Each of these articles describes using truncated versions of the entire CD4 molecule, produced by recombinant DNA techniques, to inhibit HIV-1 infection. The truncated molecule described was about 50 kilodaltons in size and comprised the extracellular segment of CD4 (i.e., the 374 amino acid portion lacking the transmembrane and cytoplasmic domains).

The so-called "soluble" forms of CD4 described in the above-mentioned articles are large protein molecules which must be prepared by recombinant DNA techniques in order to provide sufficient quantities for therapeutic use. This is a disadvantage because such methods are costly and time consuming. A further disadvantage is that such large molecules may have solubility and immunogenicity problems when used in human therapy. The present invention overcomes these disadvantages by providing relatively short HIV-1 binding peptides that can be conveniently chemically synthesized without resort to recombinant DNA techniques. The peptides of the present invention can block the binding of HIV-1 to the CD4 receptor on T4 cells. The present peptides essentially provide false targets to divert the HIV-1 and prevent T4 cell infection.

Summary of the Invention

The present invention provides isolated and substantially pure peptides comprising the following amino acid sequence (written in the standard reading frame from N-terminus to C-terminus):
Cys-Thr-Ala-Ser-Gln-Lys-Lys-Ser-Ile-Gln-Phe-His-Trp-Lys-Asn-Ser-Asn-Gln-Ile-Lys-Ile-Leu-Gly-Asn-Gln-Gly-Ser-Phe-Leu-Thr-Lys-Gly-Pro-Ser

This amino acid sequence corresponds to an amino acid sequence of the CD4 receptor protein which binds to the gp120 envelope protein of HIV-1. This sequence will hereinafter be referred to as peptide 18-51, which refers to the corresponding amino acid sequence in the CD4 molecule as deduced by Maddon et al. (Cell 42:93-104 (1985)).

Additionally, the present invention provides isolated and substantially pure peptides comprising the following amino acid sequence:
Cys-Arg-Ser-Pro-Arg-Gly-Lys-Asn-Ile-Gln-Gly-Gly-Lys-Thr-Leu-Ser-Val-Ser-Gln-Leu-Glu-Leu-Gln-Asp-Ser-Gly-Thr-Trp-Thr-Cys

This sequence will hereinafter be referred to as peptide 132-161, which also corresponds to the Maddon sequence. When used herein the word "peptides" or the phrase "peptides of the present invention" shall mean any peptide which comprises all or part of the amino acid sequence of peptide 18-51 or 132-161.

A dash at the beginning or end of an amino acid residue in the above sequences indicates a peptide bond to the next amino acid in the sequence. HIV-binding analogs, fragments, chemical derivatives and pharmaceutically acceptable salts of these sequences are within the scope of the present invention. Peptides comprising either of these sequences are capable of binding to the gp120 envelope protein of the HIV-1 virus and thereby neutralizing the virus. Thus, the peptides of the present invention may be used in a method for treating a patient suspected of being infected with the HIV-1 virus by administering one of the present peptides to the patient in an effective HIV-1 neutralizing amount. It is also contemplated that the present peptides may be used as a general immunosuppressive agent in a host by blocking binding to the CD4 receptor because the CD4 receptor may participate in other non-HIV-1 related immunoreactions in the host.

One embodiment of the peptides of the present invention is the peptide having the amino acid sequence:
$NH_2$-Trp-Lys-Asn-Ser-Asn-Gln-Ile-Lys-Ile-Leu-Gly-Asn-Gln-Gly-Ser-Phe-Leu-COOH

This peptide sequence corresponds to amino acids 30-46 on the CD4 protein and will hereinafter be referred to as peptide 30-46.

Another embodiment of the peptides of the present invention is the peptide having the amino acid sequence:
$NH_2$-Cys-Thr-Ala-Ser-Gln-Lys-Lys-Ser-Ile-Gln-Phe-His-Trp-Lys-Asn-Ser-Asn-Gln-Ile-Lys-Ile-Leu-Gly-COOH

This peptide sequence corresponds to amino acids 18-40 on the CD4 protein and will hereinafter be referred to as peptide 18-40.

Also within the scope of the present invention are antibodies specific to the peptides, pharmaceutical compositions comprising the peptides, vaccines comprising antibodies specific to the peptides, anti-idiotype antibodies which bear internal images of the peptides and diagnostic kits comprising the peptides or anti-idiotype antibodies.

Brief Description of the Figures

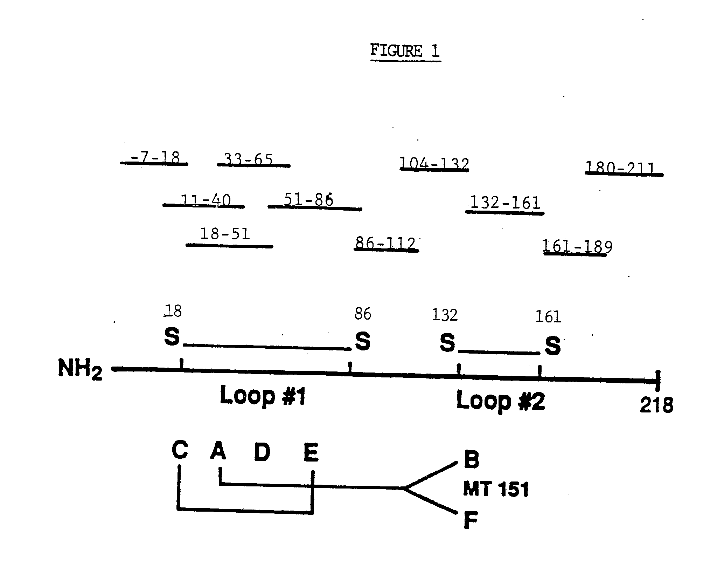

Figure 1 depicts the panel of CD4-derived synthetic peptides described in Example 1. The heavy line in the middle of this diagram is a schematic representation of the first 218 amino acids of the extracellular

3

domain of the CD4 protein, showing the positions of the first two disulfide loops of the protein as determined by Classon et al., Proc. Natl. Acad. Sci. U.S.A. 83:4499 (1986). The short lines at the top of this figure depict the family of CD4-derived peptides which were synthesized for this study. All of the peptides were synthesized on an Applied Biosystems 430A automated peptide synthesizer according to the procedures of Kent and Clark-Lewis. (Synthetic Peptides in Biology and Medicine, K. Alitalo et al., eds., Elsevier, New York, 1985, pp. 29-58) using a paramethylbenzhydrylamine resin (U.S. Biochemicals). Shown at the bottom of this figure is a summary of the results obtained with the CD4-specific monoclonal antibodies. The linear map order of the monoclonal antibodies OKT4C (C), OKT4A (A), OKT4D (D), OKT4E (E), OKT4 (B), MT 151 and OKT4F (F) is shown below the CD4 protein. The solid lines connecting the various monoclonal antibodies indicate the antibodies which displayed reciprocal binding interference patterns in the cross-competition assays.

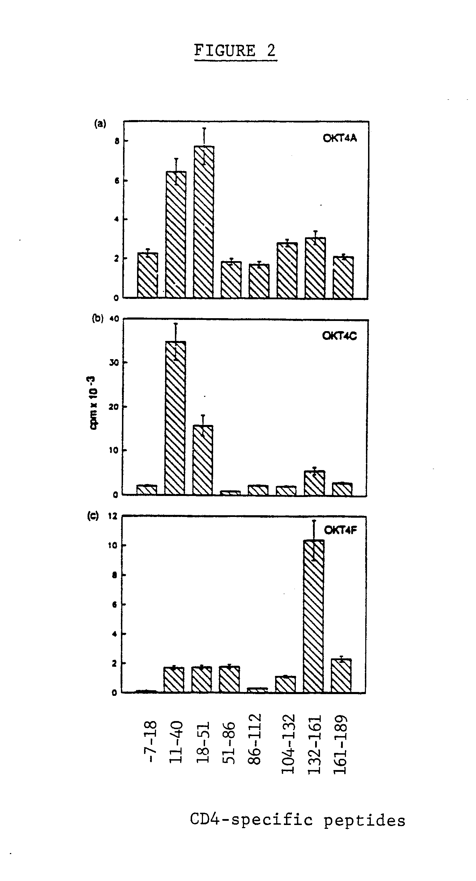

Figure 2 depicts the results of the solid phase radio-immunoassays of the synthetic peptides with the CD4-specific monoclonal antibodies OKT4A, OKT4C and OKT4F. The data shown here are the average of triplicate assays (mean deviation shown by bars; background cpm subtracted). The 20 micrograms of peptide was absorbed to the bottom of 96 well microtiter plates. After washing (tri-buffered saline with 0.1% calf serum), the plates were blocked with 0.1% swine skin gelatin (Sigma) before the addition of the monoclonal antibody (20 micrograms/ml), according to the procedure of Emini et al. (Nature 304:699 (1983)) except that the binding of the monoclonal antibodies was monitored with an $^{125}$I-labeled goat anti-mouse antibody (ICN Biochemicals).

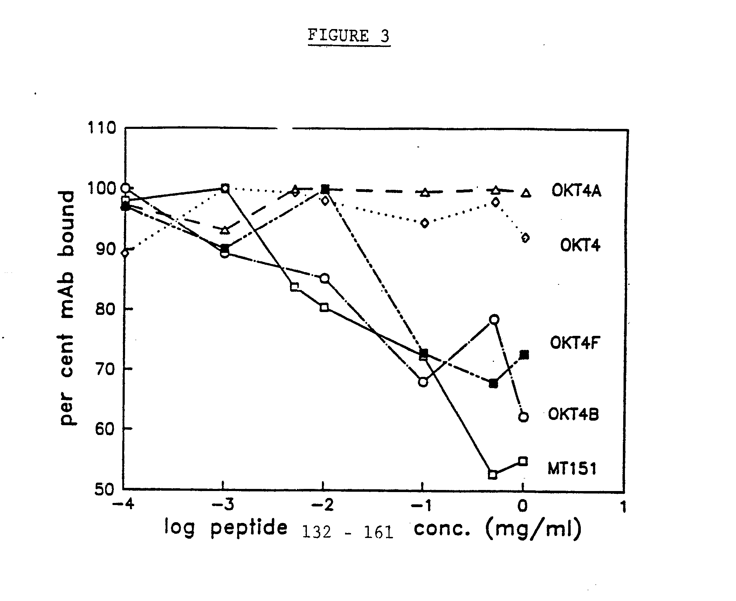

Figure 3 depicts the results of the CD4-specific monoclonal antibody competitions with peptide 132-161. For this assay, the following CD4-specific monoclonal antibodies were used: OKT4A (Δ) OKT4 ( ◇ ), OKT4F (-□-), OK4B (-O-) and MT151 (-□-). The y-axis shows the percentage of antibody bound at various concentrations of the 132-161 peptide. The assay was performed using 8 x 10$^5$ cells/well in a concentration dependent manner.

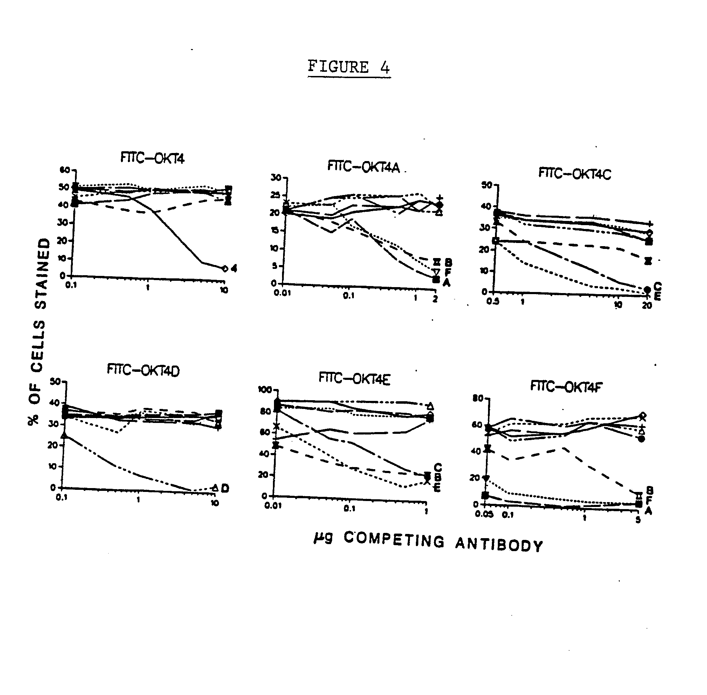

Figure 4 depicts the results of competition assays between FITC labeled monoclonal antibodies.

Detailed Description of the Invention

The present invention is based on the elucidation of the HIV-1 binding sites on the CD4 protein and the determination of the amino acid sequences of those portions of the CD4 protein to which HIV-1 binds. It is believed that two binding sites exist on the CD4 molecule, one binding site on each side of an invagination. Knowledge of these amino acid sequences has enabled the synthesis of peptides of the present invention which are capable of binding to HIV-1 and thereby blocking the attachment of HIV-1 to the CD4 protein. As is readily apparent, these peptides may be used in the prevention of HIV-1 infection or therapy for individuals who are already infected by HIV-1. The present peptides may also block the binding of other HIV related viruses (such as HIV-2) if such related viruses bear envelope proteins similar to gp120. The present peptides are relatively short in length and therefore they are easily synthesized by chemical means. Such synthetic peptides have many advantages over the use of the entire CD4 protein or large portions of the CD4 protein as has been described in the literature. For instance, the large portions of the CD4 protein cannot conveniently be made by synthetic techniques and must be made by recombinant DNA techniques, which are expensive and time consuming. Additionally, the larger CD4 proteins may present solubility and immunogenicity problems when introduced into a patient. Short synthetic peptides are much more soluble and less immunogenic than larger proteins.

As used herein, "peptide" refers to a linear series of no more than about 50 amino acid residues connected to one another by peptide bonds between the alpha-amino groups and carboxy groups of adjacent amino acid residues. When used herein, "protein" refers to a linear series of greater than 50 amino acid residues connected one to the other as in a peptide. The term "synthetic peptide" means a chemically derived chain of amino acid residues linked together by peptide bonds that is free of naturally occuring proteins and fragments thereof.

The three-letter symbols used to represent the amino acid residues in the peptides of the present invention are those symbols commonly used in the art. The amino acid residues are preferred to be in the "L" isomeric form. However, residues in the "D" isomeric form may be substituted for any L-amino acid, as long as the desired functional property of HIV-1 binding is retained by the peptide. NH$_2$ refers to a free amino group and - COOH refers to a free carboxyl group. The three-letter symbols used herein refer to the following amino acids: Cys is cysteine; Ser is serine; Ile is isoleucine; Gln is glutamine; Phe is phenylalanine; His is histidine; Trp is tryptophan; Lys is lysine; Asn is asparagine; Leu is leucine; Gly is glycine; Thr is threonine; Asp is aspartic acid; Arg is arginine; Val is valine; and Ala is alanine.

Peptides of the present invention include any analog, fragment or chemical derivative of peptide 18-51 or peptide 132-161 capable of binding to the gp120 binding site on HIV-1. The term "analog" refers to any peptide having a substantially identical amino acid sequence to peptide 18-51 or peptide 132-161 in which one or more amino acids have been substituted with chemically similar amino acids. For instance, one polar amino acid, such as glycine or serine, may be substituted for another polar amino acid; or one acidic amino acid, such as aspartic acid may be substituted for another acidic amino acid, such as glutamic acid; or a basic amino acid, such as lysine, arginine or histidine may be substituted for another basic amino acid; or a non-polar amino acid, such as alanine, leucine or isoleucine may be substituted for another non-polar amino acid.

The term "analog" shall also include any peptide which has one or more amino acids deleted from or added

4

to peptide 18-51 or peptide 132-161, but which still retains a substantial amino acid sequence homology to these peptides. A substantial sequence homology is any homology greater than 50%. The term "fragment" shall refer to any shorter version of the peptides identified herein having at least five amino acid residues, wherein the fragment is capable of binding to the gp120 envelope protein of HIV-1 and blocking HIV-1 binding to the CD4 receptor.

The term "chemical derivative" refers to any peptide derived from peptide 18-51 or peptide 132-161 and in which one or more amino acids have been chemically derivatized by reaction of the functional side groups of the amino acids residues present in the peptide. Thus, a "chemical derivative" is a peptide that is derived from the sequences or peptides identified herein by one or more chemical steps. Such derivatized molecules include, for example, those molecules in which free amino groups have been derivatized to form amine hydrochlorides, p-toluene sulfonyl groups, carbobenzoxy groups, t-butyloxycarbonyl groups, thiourethane-type derivatives, trifluoroacetyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those peptides which contain one or more naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine; and ornithine may be substituted for lysine.

The peptides of the present invention may be prepared by any of the following known techniques. Conveniently, the peptides may be prepared using the solid-phase synthetic technique initially described by Merrifield, in J. Am. Chem. Soc. 85:2149-2154 (1963). Other peptide synthesis techniques may be found, for example, in M. Bodanszky et al., Peptide Synthesis, John Wiley & Sons, 2d Ed., (1976) as well as in other reference works known to those skilled in the art. A summary of peptide synthesis techniques may be found in J. Stuart and J. D. Young, Solid Phase Peptide Synthesis, Pierce Chemical Company, Rockford, IL (1984). The synthesis of peptides by solution methods may also be used, as described in The Proteins, Vol. II, 3d Ed., Neurath, H. et al., Eds., p. 105-237, Academic Press, New York, NY (1976). Appropriate protective groups for use in such syntheses will be found in the above texts as well as in J .F. W. McOmie, Protective Groups in Organic Chemistry, Plenum Press, New York, NY (1973). Of course, the present peptides may also be prepared by recombinant DNA techniques, although such methods are not preferred.

In general, these synthetic methods comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as an example, the protected or derivatized amino acid is attached to an inert solid support through its unprotected carboxyl or amino group. The protecting group of the amino or carboxyl group is then selectively removed and the next amino acid in the sequence having the complementary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence any remaining terminal and side group protecting groups (and solid support) are removed sequentially or concurrently, to provide the final peptide.

The peptides of the present invention generally contain at least five amino acid residues and up to fifty amino acid residues, preferably 5-35 amino acid residues. These peptides may be linked to an additional sequence of amino acids at either or both the N-terminus and C-terminus, wherein the additional sequences are from 1-100 amino acids in length. Such additional amino acid sequences, or linker sequences, can be conveniently affixed to a detectable label or solid matrix, or carrier. Labels, solid matrices and carriers that can be used with peptides of the present invention are described below. Typical amino acid residues used for linking are tyrosine, cysteine, lysine, glutamic acid and aspartic acid, or the like.

The small peptides of the present invention are generally antigenic rather than immunogenic. That is, the peptides may be bound by an antibody specific to them (antigenic) but they may not induce antibody production in a host (immunogenic). Standard techniques that are well known in the art may be used to render the present peptides immunogenic if necessary (that is, if it is not immunogenic in the host as synthesized). One such method is to bind the peptide to an antigenic carrier as a conjugate and then inject the conjugate in an effective amount as an immunogenic innoculum into a host.

Any peptide of the present invention may be used in the form of a pharmaceutically acceptable salt. Suitable acids which are capable of forming salts with the peptides of the present invention include inorganic acids such as hydrochloric acid, hydrobromic acid, perchloric acid, nitric acid, thiocyanic acid, sulfuric acid, phosphoric acid and the like; and organic acids such as formic acid, acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like.

Suitable bases capable of forming salts with the peptides of the present invention include inorganic bases such as sodium hydroxide, ammonium hydroxide, potassium hydroxide and the like; and organic bases such as mono-, di- and tri-alkyl and aryl amines (e.g. triethylamine, diisopropyl amine, methyl amine, dimethyl amine and the like) and optionally substituted ethanolamines (e.g. ethanolamine, diethanolamine and the like).

For use in a method of treating HIV-1 infection, the peptides of the present invention may be present in a pharmaceutical composition in an effective HIV-1 binding amount in admixture with a pharmaceutically-acceptable carrier. The pharmaceutical composition may be compounded according to conventional pharmaceutical formulation techniques. The carrier may take a wide variety of forms depending on the form of preparation desired for administration, e.g. sublingual, rectal, nasal, oral or parenteral. Compositions for oral dosage form may include any of the usual pharmaceutical media, such as, for example, water, oils, alcohols, flavoring agents, preservatives, coloring agents and the like in the case of oral liquid preparations (e.g. suspensions, elixirs and solutions) or carriers such as starches, sugars, diluents, granulating agents, lubricants, binders, disintegrating agents, and the like in the case of oral solid preparations (e.g. powders, capsules and tablets). Controlled release forms may also be used. Because of their ease in administration, tablets and capsules represent the most advantageous oral dosage unit form, in which case solid pharmaceutical carriers are obviously employed. If, desired, tablets may be sugar coated or enteric coated by standard techniques.

For compositions to be administered parenterally, the carrier will usually comprise sterile water, although other ingredients to aid solubility or for preservation purposes may be included. Injectable suspensions may also be prepared, in which case appropriate liquid carriers, suspending agents and the like may be employed. The parenteral routes of administration may be intravenous injection, intramuscular injection or subcutaneous injection.

The pharmaceutical compositions of the present invention may be used in a method for treating a patient suspected of being infected with HIV-1. The method comprises administering to the patient an effective HIV-1 neutralizing amount of the pharmaceutical composition. Upon administration, the peptides in the composition will bind to the gp120 of HIV-1 and prevent the HIV-1 from binding to the CD4 receptor. Such interruption of the binding process will prevent the T4 cell from being infected by the HIV-1 and will thereby prevent the usual immunosuppression observed in an HIV-1 infected patient.

The present invention also contemplates antibodies which are specific for the peptides of the present invention. It is believed that such antibodies will also prevent the binding of the HIV-1 to the CD4 receptor of the T4 cell. Such antibodies will bind to the receptor site on the CD4 protein and thereby competitively inhibit the binding of the HIV-1 to that same site. Such antibodies may be polyclonal or monoclonal in nature; however, it is preferred that the antibodies be monoclonal.

Polyclonal antibodies may be prepared according to methods well known in the art, such as those described in Benedict, A. A., et al., "Production of Antiserum", Methods in Immunology 1:197-306 (1967). It is preferred that monoclonal antibodies be used in the present method because they are specific for a single epitope and therefore provide more reproducible results. Methods of producing monoclonal antibodies are well known in the art, such as the well known Kohler and Milstein method. This method as well as variations thereof may be obtained by reference to Yelton, D. E. et al., Ann. Rev. of Biochem. (1981) 50:657-80.

The present invention also contemplates an anti-idiotype antibody that bears an internal image of a peptide of the present invention and which is thereby capable of binding to the gp120 envelope protein of HIV-1. Such an anti-idiotype antibody may be used in any therapeutic or diagnostic format where the formation of an HIV-1-containing immunoreaction product is desired.

By way of background, a brief description of anti-idiotype antibodies is provided. The immune system of an individual is capable of producing millions of different types of antibodies. Each antibody can in turn be the target of other antibodies that recognize its unique molecular characteristics. By means of such antibody-antibody reactions, the immune system interacts with itself. These interactions are known as idiotype-anti-idiotype reactions. In such reactions, an initial antibody (Ab-1) that is isolated from an animal serum and then injected into another animal induces the formation of a second antibody (Ab-2). The Ab-2 is specific for and binds to the Ab-1 and is said to recognize Ab-1's individuality. The unique antigenic determinants on the Ab-1 are referred to as idiotypes. The Ab-2 produced in response to the idiotype is referred to as an anti-idiotype antibody. Thus, an anti-idiotype antibody may be defined as an antibody that is capable of reacting with an Ab-1 elicited by a single antigen. For more complete background information on anti-idiotype antibodies, see Kennedy, R.C. et al. "Anti-Idiotypes and Immunity," Scientic American, pages 48-56 (July 1986).

Idiotopes are antigenic determinants (epitopes) expressed by an antibody molecule. Internal image idiotopes are antigenic determinants expressed by an antibody molecule that are immunologically similar or identical to antigenic determinants found on an antigen that is foreign or external to the immune system. The operational theory explaining the immunologic similarity of an internal image idiotype and its corresponding external antigenic determinant is that the internal image idiotype is a conformational homolog of the external antigenic determinant. Because they are conformationally homologous, an internal image idiotype and its corresponding external antigenic determinant are immunologically cross reactive, i.e., an antibody induced by one will immunoreact with the other. Therefore, an antibody expressing an internal image idiotype corresponding to a peptide of the present invention (an external antigenic determinant) can be substituted for that peptide in blocking the binding site on the HIV-1 virus.

The production of an antibody expressing an internal image corresponding to an antigenic determinant on an infectious agent requires finding or producing an immunogenic "template" that can induce the internal image bearing antibody. Templates for producing internal image antibodies are immunogenic molecules having at least a portion of their conformation that is complimentary to or the mirror image of the antigenic determinant or interest. For infectious agents, template-bearing molecules used by the art to induce internal image-bearing antibodies are proteins that bind the infectious agent of interest. Such proteins include antibody molecules whose antibody combining sites immunoreact with the infectious agent of interest and cell service receptor proteins that bind the infectious agent. In either case, the critical feature of the binding protein is that portion of its structure that interacts with the infectious agent because it is only that portion that can act as template. Thus, the antibodies of the present invention specific for the peptides of the present invention can be used as immunogens to induce an anti-idiotypic antibody, i.e, an antibody that immunoreacts with an idiotope. The anti-idiotypic antibody will have that idiotope that can block the CD4 binding site of the HIV-1.

It is preferred that the anti-idiotype antibody be raised against a monoclonal antibody. The preparation of an anti-idiotypic antibody against a monoclonal antibody combining site is well known in the art. For example, see Staudt et al., J. Exp. Med., 157:687-704 (1983); and Regan et al., J. Virol. 48:660-666 (1983). Briefly, to produce an anti-idiotypic antibody composition of this invention, a laboratory mammal is inoculated with an immunologically-effective amount of a peptide of the present invention, typically as present in a vaccine of the present invention. The anti-peptide homolog antibody molecules thereby induced are then collected from the mammal and those immunospecific for both the peptide paratope and the binding site on the gp120 are isolated to the extent desired by well known techniques such as, for example, by immunoaffinity chromatography.

Monoclonal anti-idiotypic antibody compositions are contemplated by the present invention. A monoclonal anti-idiotypic antibody composition contains, within detectable limits, only one species of antibody combining site capable of effectively immunologically binding a peptide homolog paratope and HIV-1. Thus, a monoclonal anti-idiotypic antibody composition of the present invention typically displays a single binding affinity for the peptide.

It is contemplated that the antibodies of the present invention can block the binding site on the CD4 receptor so that the HIV-1 virus cannot infect the T4 cell. Therefore, these antibodies may be used in a vaccine to confer protection on a patient against HIV-1. Additionally, such a vaccine will cause the patient to produce anti-idiotype antibodies in response to the initial antibody and thereby will confer upon the patient active immunity against the HIV-1 virus because the anti-idiotype antibody will bind to the HIV-1 and block its binding site. Accordingly, a vaccine of the present invention comprises an effective immunizing amount of an antibody of the present invention and a pharmaceutically-acceptable carrier. It is preferred that the antibody used in the vaccine is a monoclonal antibody. "Humanized" monoclonal antibodies are preferred as they will be less immunogenic than a murine monoclonal, for example.

The antibody molecule may be operatively linked to an immunogenic carrier. As used herein, the term "operatively linked" means that the antibody and carrier protein are physically associated by a linking means that does not interfere with the ability of either of the linking groups to function as described. Because immunogenic carriers are typically proteins themselves, the techniques of protein conjugation or coupling through activated functional groups is particularly applicable. For review of those techniques, see Aurameas et al., Scand. J. Immunol., Vol. 8, Supp. 1, pp. 7-23 (1978). See, also, U.S. Patent Nos. 4,493,795 and 4,671,958. Useful immunogenic carriers are well known in the art and are generally large proteins. Exemplary of such carriers are keyhole limpet hemocyanin (KLH); edestine, thyroglobulin; albumins, such as bovine serum albumin (BSA) or human serum albumin (HSA); red blood cells, such as sheep erythrocytes (SRBC); tetanus toxoid; cholera toxoid; and polyamino acids, such as poly(d-lycine:d-glutamic acid) and the like.

The vaccine may also contain an immunopotentiator, which is a molecular entity that stimulates the maturation, differentiation and function of B and/or T-lymphocytes. Immunopotentiators are well known in the art and include T-cell stimulating polypeptides such as those described in U.S. Patent No. 4,426,324 and the C8-substituted guanine nucleosides described by Goodman et al., J. Immunol., 135:3284-3288 (1985) and U.S. Patent No. 4,643,992. The phrases "suitable for human use" and "pharmaceutically-acceptable" refer to molecular entities and compositions that do not produce an allergic or similar untoward reaction when administered to a human.

The preparation of a vaccine that contains an antibody molecule as an active ingredient is well understood in the art. Typically, such vaccines are prepared as injectibles, either as liquid solutions or suspensions; solid forms suitable for solution in or suspension in liquid prior to injection may also be prepared. The preparation may also be emulsified.

The active immunogenic ingredient may be dissolved, dispersed or admixed in an excipient that is pharmaceutically-acceptable and compatible with the active ingredient as is well known. Suitable excipients are, for example, water, saline, phosphate buffered saline (PBS), dextrose, glycerol, ethanol or the like and combinations thereof. In addition, if desired, the vaccine may contain minor amounts of auxiliary substances such as wetting or emulsifying agents, pH buffering agents or adjuvants which enhance the effectiveness of the vaccine.

The vaccines may conventionally be administered parenterally, by either intravenous, subcutaneous, or intramuscular injection. Additional formulations which are suitable for other modes of administration include suppositories and, in some cases, oral formulations. For suppositories, traditional binders and carriers may include, for example, polyalkylene glycols or triglycerides. Oral formulations include such normally employed excipients as, for example, pharmaceutical grades of mannitol, lactose, starch, magnesium stearate, sodium saccharin, cellulose, magnesium carbonate and the like. Compositions may take the form of solutions, suspensions, tablets, pills, capsules, sustained release formulations or powders. The anti-peptide antibody can be formulated into a vaccine as a neutral or salt form. Pharmaceutically-acceptable salts include those listed above.

The vaccines may be administered in a manner compatible with the dosage formulation, and in such amount as will be therapeutically effective and immunogenic. The quantity to be administered depends on the subject to the treated, capacity of the subject's immune system to synthesize the antibodies and the degree of protection desired. Precise amounts of active ingredient required to be administered depend on the judgment of the practitioner and are peculiar to each individual.

The vaccine may also include an adjuvant as part of the excipient. Adjuvants such as complete Freund's adjuvant (CFA) or, incomplete Freund's adjuvant (IFA) for use in laboratory animals are well known in the art. Pharmaceutically-acceptable adjuvants such as alum may also be used.

The present invention also contemplates pharmaceutical compositions which comprise one of the peptides of the present invention and an antiviral agent as a form of combination therapy for HIV-1 infection. The peptide and antiviral agent may be in separate molecular form or they may be linked or conjugated by a chemical bond. In the case where the peptide and antiviral agent are linked, the resulting conjugated molecule would serve as a means for specific targeted delivery of the antiviral agent to the HIV-1. It is also envisioned that the peptide and antiviral agent may be administered separately in a combination therapy. Fischer et al. (Nature 331: 76-78 (1988)) suggested the use of an antiviral therapy for HIV-1 which involves administering to a patient soluble CD4 protein in combination with an antiviral drug that blocks reverse transcriptase.

The antiviral agent may be any agent that is capable of blocking one or more stages in the life cycle of HIV-1. One class of such an antiviral agent are the reverse transcriptase inhibitors, which are capable of inhibiting the function of the enzyme reverse transcriptase present in all retroviruses. Many of the reverse transcriptase inhibitors presently in use are nucleoside analogs or derivatives, such as 3'-azido-3'-deoxythimidine (AZT); dideoxycytidine; dideoxyadenosine and other nucleoside analogs, such as acyclovir, ribavirin, and vidarabine. European Patent Application Publication No. EP 196,185 describes the use of AZT for treating AIDS. These nucleoside analogs interupt the reverse transcriptase as it assembles the viral DNA destined to become the pro-virus of HIV-1. The antiviral drugs used for this purpose are typically chemical analogs of the nucleosides that form the sub-units of DNA. When the analog is supplied to an infected cell, reverse transcriptase will incorporate it into a growing DNA chain. Because the analog lacks the correct attachment point for the next sub-unit, however, the chain is terminated. The truncated DNA cannot integrate itself into the chromosomes or provide the basis for viral replication, and, so, the spread of infection is halted.

Other anti-viral agents that may be useful are those which can block the formation of the HIV-1 envelope protein, such as castanospermine; any agent which can interfere with the assembly of new virus components in infected cells; or any agent which can interfere with the "budding" of completed virus particles from infected cells.

As previously mentioned, it is contemplated that the peptides of the present invention may be used as immunosuppressive agents by mimicking the CD4 protein. The basis for this application of the present peptides is that the CD4 protein is thought to interact with class II MHC proteins expressed by antigen-bearing target cells. This presumed interaction between class II MHC proteins and T4 may be essential for the appropriate function of the T cell. See, Biddison, W.E. et al., J. Immunology 131:152 (1983); and Maddon, P.J. et al., Cell 47:333-348 (1985). Antibodies binding to the CD4 molecule or the class II molecule interfere sufficiently with the cellular interaction to inhibit in vitro immune responses.

It is believed that, like antibodies, peptides are capable of blocking the CD4-class II MHC protein interaction and can interfere with the immune response to such an extent that such peptides can act as immunosuppressive agents. The T4 lymphocyte is the focal and critical cell involved directly or indirectly in the induction of most immunologic functions by affecting a wide array of functions of multiple limbs of the immune response as well as certain nonlymphoid cell functions. This is effected generally by the secretion of a variety of soluble factors (e.g., lymphokines such as interleukin-2) that have trophic or inductive effects (or both) on the cells in question. Examples of some of the functions that the T4 lymphocyte carrys out in the immune system are: secretion of growth and differentiation factors for lymphoid cells; secretion of hematopoietic colony stimulating factors; secretion of factors which induce non-lymphoid cell function; induction of B cell function; induction of suppressor cell function; induction of killer T cell function; induction of cyotoxic T cell function; and activation of macrophages.

Thus, the peptides of the present invention may find utility as immunosuppressive agents by interfering with or inhibiting the immune response. Such an effect may be desirable in treating those conditions in which it is desirable to suppress the immune system. Such conditions include autoimmune diseases, allergy, rejection of allografts, delayed-type-hypersensitivity reaction and graft-vs.-host reaction.

It is also contemplated that the peptides of the present invention may find use as diagnostic agents for detecting the presence of HIV-1 because these peptides are capable of specifically binding to the virus. Such a diagnostic system in kit form includes, in an amount sufficient for at least one assay, a peptide or anti-idiotype antibody composition of the present invention, as a packaged reagent. Instructions for use of the packaged reagent are also typically included in such a kit. The peptide or anti-idiotypic antibody may be labeled with a detection means such as a radioactive, fluorescent or enzymatic label. Such a detection means allows for the detection of the peptide or anti-idiotype antibody when it is bound to HIV-1. The peptide or anti-idiotype antibody may be used as a "capture ligand" bound to a solid matrix. In such a case, a labelled antibody specific for HIV-1 is also used to detect the virus. Alternatively, the peptide or anti-idiotype antibody may be used as the "detector ligand" and the anti-HIV-1 antibody used as the capture ligand.

As used herein, the term "packaged" refers to a solid matrix for materials such as glass, plastics, paper, foil and the like capable of holding, within fixed limits, a peptide or anti-idiotypic antibody composition of the present invention. Thus, for example, a package can be a glass vial used to contain milligram quantities of a contemplated peptide or anti-idiotypic antibody or it can be a microtiter plate well to which microgram quantities of a contemplated peptide or anti-idiotypic antibody have been operatively affixed, i.e., linked so as to be capable of being bound by HIV-1. "Instructions for use" typically include a tangible expression describing the reagent concentration or at least one assay method parameter such as the relative amounts of reagent sample to be admixed, maintenance time periods for reagent/sample admixtures, temperature, buffer conditions and the like.

As used herein, the terms "label" and "detector means" and their various grammatical forms refer to single atoms and molecules that are either directly or indirectly involved in the production of a detectable signal to indicate the presence of a complex. The word "complex" as used herein refers to the product of the specific binding reactions such as the peptide-HIV-1 or anti-idiotype-HIV-1 reaction. Any label or detection means can be linked to or incorporated into the peptide or anti-idiotypic antibody of the present invention, or used separately, and those atoms or molecules can be used alone or in conjunction with additional reagents. Such labels are themselves well known in clinical diagnostic chemistry and constitute a part of this invention only insofar as they are utilized with otherwise novel peptides and anti-idiotype antibodies.

The labeling means can be a fluorescent labeling agent that chemically binds to antibodies or antigens without denaturing them to form a fluorochrome (dye) that is a useful immunofluorescent tracer. Suitable fluorescent labeling agents are fluorochromes, such as fluorescein isocyanate (FIC), fluorescein isothiocyanate (FITC) and the like. A description of immunofluorescence analysis techniques is found in DeLuca, "Immunofluorescence Analysis", in Antibody as Tool, Marchalonis et al., Eds., John Wiley & Sons Limited, pp. 189-231 (1982).

In preferred embodiments, the indicating group is an enzyme, such as horseradish peroxidase (HRP), glucose oxidase or the like. In such cases where the principal indicating group is an enzyme such as HRP or glucose oxidase, additional reagents are required to visualize the fact that a binder complex has formed. Such additional reagents for HRP include hydrogen peroxide and an oxidation dye-precursor, such as diaminobenzidine.

Radioactive elements are also useful labeling agents and are used illustratively herein. An exemplary radio-labeling agent is a radioactive element that produces gamma ray emissions. Elements which themselves emit gamma rays, such as $^{124}$I, $^{125}$I, $^{128}$I, $^{132}$I and $^{51}$Cr represent one class of gamma ray emission-producing radioactive element indicating groups. Particularly preferred is [125]I. Another group of useful labeling means are those elements such as $^{11}$C, $^{18}$F, $^{15}$O and $^{13}$N which themselves emit positrons. The positrons so emitted produce gamma rays upon encounters with electrons present in the test solution.

The linking of labels, i.e., labeling of proteins, is well known in the art. For instance, anti-idiotypic antibody molecules produced by a hybridoma can be labeled by metabolic incorporation of radioisotope-containing amino acids provided as a component in the culture medium. See, for example, Galfre et al., Meth. Enzymol, 73:3-46 (1981). The techniques of protein conjugation or couplings through activated functional groups are particularly applicable.

The diagnostic kits of the present invention may be used in a solid phase format to detect the presence of a quantity of HIV-1 in a body fluid sample such as serum, plasma or urine. In the solid phase assay, the peptide or anti-idiotype antibody may be bound to a solid phase and then contacted with the sample containing the HIV-1. The HIV-1 in the sample will bind to the peptide or anti-idiotype antibody. Thus, in preferred embodiments, a peptide or anti-idiotype antibody molecule composition of the present invention may be affixed to a solid support to form a solid support that comprises a package in the subject diagnostic system. The reagent is typically affixed to the solid matrix by adsorption from an aqueous medium although other modes of affixation, well known to those skilled in the art, can be used. Useful solid matrices are also well known in the art. Such materials are water-insoluble and include the cross-linked dextran available under the trademark SEPHADEX® from Pharmacia Fine Chemicals; agarose; beads of polystyrene; polyvinylchloride; polystyrene; cross-linked polyacrylamide; nitrocellulose- or nylon-based webs such as sheets, strips or paddles; or tubes, plates or the wells of microtiter plates such as those made from polystyrene or polyvinylchloride.

The peptide or anti-idiotype antibody of the present invention can be used in the diagnostic system in solution form, as a liquid dispersion, or as a substantially dry powder, e.g. in lyophilized form. Where the indicating means is an enzyme, the enzyme substrate can also be provided in a separate package of the system. A solid support such as the before-described microtiter plate and one or more buffers can also be included as separately packaged elements in this diagnostic assay system.

The packaging materials discussed herein in relation to diagnostic systems are those customarily utilized in diagnostic systems. Such materials include glass and plastic (e.g. polyethylene, polypropylene and polycarbonate) bottles, vials, plastic and plastic-foil laminated envelopes and the like.

The following example is presented to illustrate the subject invention. The invention is not to be considered limited by this example but only by the appended claims.

Example 1

The structure for the HIV-1 binding site on the CD4 protein was identified by epitope mapping using a family of eight functionally distinct CD4-specific monoclonal antibodies in conjunction with a panel of large CD4-derived synthetic peptides. All of the seven epitopes that were located reside within two immunoglobulin-like disulfide loops situated between residues 1 and 161 of the CD4 protein. The CD4-specific monoclonal antibody OKT4A, a potent inhibitor of HIV-1 binding, recognized a site between residues 25 and 40 on the CD4 protein. By analogy to other members of the immunoglobulin superfamily of proteins, this particular region has been predicted to exist as a protruding loop. A synthetic analog of this loop (residues 18 to 51) showed a concentration-dependent inhibition of HIV-1-induced cell fusion. It is believed that a loop extending from residues 30-46 or 18-40 of the CD4 protein may be the actual binding site for HIV-1.

A panel of eight CD4-specific monoclonal antibodies (Table 1) was used in the studies described below. Antibodies MT-151, OKT4F and OKT4A are the most potent inhibitors of HIV-1 binding to the CD4 protein, while OKT4D and OKT4E show intermediate inhibitory effects, OKT4B shows only a weak effect, and OKT4 and OKT4C show no effect on virus binding. McDougal et al., J. Immunol. 137:2937 (1986); and Sattentau et al., Science 234:1120 (1986). Sattentau et al. (supra) suggested that two independent binding domains can be discerned by this panel of CD4-specific monoclonal antibodies, one defined by OKT4A and the other by MT151.

TABLE 1

| Antibody | Anti-CD4 React. | Competing Monoclonals | | Syncytia Inhib. | Solid-Phase Peptide Reactivity | Peptides Competing for CD4 Binding |
|---|---|---|---|---|---|---|
| | | RIA | FITC | | | |
| anti-18-51 | + | A | n.d. | | 18-51 | |
| anti-33-65 | + | n.d. | D...A,E | | 33-65 | |
| anti-51-86 | + | E...D | E | | 51-86 | |
| anti-104-139 | - | n.d. | n.d. | | 104-132 | |
| anti-132-161 | + | B,MT151...F | B | | 132-161 | |
| anti-180-211 | + | none | none | | 180-211 | |
| OKT4 | + | n.d. | none | - | - | - |
| OKT4A | + | n.d. | F,B | strong | 18-51,11-40 | n.d. |
| OKT4B | + | n.d. | A,F | weak | - | 132-161 |
| OKT4C | + | n.d. | E | - | 11-40,(18-51) | n.d. |
| OKT4D | + | n.d. | none | interm. | - | - |
| OKT4E | + | n.d. | C | interm. | - | - |
| OKT4F | + | n.d. | A,B. | strong | 132-161 | 132-161 |
| MT151 | + | n.d. | n.d. | strong | - | 132-161 |

Antipeptide antisera were prepared with an initial intradermal inoculation of keyhole limpet hemocyanin (KLH)-linked peptide (1 mg) mixed 1:1 with Freund's complete adjuvant. Three weeks later, rabbits received an intramuscular inoculation of 1 mg of free peptide mixed 1:1 with Freund's incomplete adjuvant. Following this, the animals were inoculated in the same manner at two week intervals. The antipeptide titers were measured at 10 weeks post-inoculation exactly as described by Emini et al. (Nature 304:699 (1983)). The ability of antipeptide antisera to bind to native CD4 protein was assayed as described in the legend to Fig. 3, except that an $^{125}$I-labeled goat anti-rabbit IgG second antibody (ICN Biochemicals) or an FITC-labeled donkey anti-rabbit IgG second antibody (Jackson Immunologicals) was used.

Competition between CD4-specific monoclonal antibodies and a 1:100 dilution of the rabbit antipeptide antisera was performed as described herein. The letter shown in either the RIA or FITC columns of Table 1 refer to the last letter of the monoclonal antibody for which strong competition (>80% reduction of antipeptide-CD4 binding) was observed, e.g. OKT4A = A. The monoclonal antibodies which are preceded by three dots were observed to significantly compete with the peptide antisera (>40% binding reduction), but to a lesser extent than those antibodies which appear before the three dots. The results shown for syncytia inhibition with respect to the CD4-specific monoclonal antibodies were taken from McDougal et al. (J. Immunol. 137:2937 (1986)) and Sattentau et al. (Science 234:1120 (1986)). The solid phase RIA results with the antipeptide sera were not assayed for cross-reactivity, e.g. whether or not anti-18-51 cross-reacts with the peptide 33-65.

To map the epitopes of the family of CD4-specific monoclonal antibodies, we synthesized a panel of CD4-derived peptides (see Fig. 1). We have found that longer peptides (approximately 30 amino acids in length) give more consistent results in direct binding assays than shorter peptides (6-12 amino acids in length) and that antibodies to the longer peptides cross-react better with the native target protein. We, therefore, used peptides of about 30 amino acids in length. The CD4-specific monoclonal antibodies were directly assayed for their ability to bind to each synthetic peptide (Fig. 2). Only three of the monoclonal antibodies consistently recognized any of the CD4-derived peptides in the solid-phase RIA. OKT4C and OKT4F both gave reproducible strong signals in the assay, whereas OKT4A, although consistently positive, gave a much weaker signal. OKT4C reacted positively with peptides 11-40 and 18-51. The reaction with peptide 11-40 was consistently the stronger of the two. OKT4A reacted equally well with peptides 11-40 and 18-51. This can be consistently interpreted as follows. Both OKT4C and OKT4A map to the amino-terminal portion of the first disulfide loop of the CD4 protein (see Fig. 1), with OKT4C binding near the amino terminus of 18-51 and to the middle of 11-40, while OKT4A binds toward the middle of both peptides. This implies that OKT4C binds to a region slightly amino terminal to the OKT4A epitope. OKT4F, which inhibits HIV from binding to the CD4, bound only to peptide 132-161. Thus, this antibody interacts with residues within the second disulfide loop of the CD4 protein (see Fig. 1).

Although neither MT151 nor OKT4B recognized any of the CD4-derived synthetic peptides in the solid-phase RIA, they specifically recognized one peptide in a solution competition binding assay. Each monoclonal antibody was titrated against a constant number of CD4(+) CEM cells (8 x $10^5$ cells/well). An antibody concentration (8 micrograms/ml) was selected that was in the pre-saturation portion of the binding curve and the peptide 132-161 was used to compete (in a concentration-dependent manner) for the binding of the monoclonal antibody to native CD4 presented on the T-helper lymphocytes (CEM cells). With this assay, we were able to map the binding sites of MT151 and OKT4B and confirm that of OKT4F (Fig. 3). From the data, we conclude that OKT4B, OKT4F and MT151 all bind to the second disulfide loop (132-161).

We then inoculated CD4-derived peptides (18-51, 33-65, 51-86, 104-132, 132-161 and 180-211) into female New Zealand white rabbits. All of the peptides used in this study elicited high titers of antibody to the peptides and, except for the antiserum to peptide 104-132, bound specifically to CD4(+) CEM cells. (The binding of the rabbit antibodies was monitored either with an $^{125}$I- or FITC-labeled second antibody). Saturating amounts (20 micrograms/ml) of the CD4-specific monoclonal antibodies were used to competitively inhibit the binding of each of the rabbit antibodies to the CD4(+) cells. The results of these competitions are shown in Table 1. Antibody to peptide 180-211, bound to the native CD4 protein but was not inhibited by any of the CD4-specific monoclonal antibodies. Prebinding of OKT4A to the CD4 protein abolished the binding of the antibody to peptide 18-51, consistent with the solid-phase RIA direct binding assay data. OKT4D strongly inhibited the binding of the anti-33-65 antibodies in the fluorescence (FITC) assay. However, the anti-33-65 antisera did not show reproducible binding to the CD4 protein in the ($^{125}$I) radio-immunoassays. Preincubation of the CD4(+) cells with OKT4E inhibited the binding of the rabbit anti-51-86 antibodies. In the radio-immunoassays, OKT4E also inhibited the binding of the anti-51-86 antibodies but to a lesser extent. These data suggest that OKT4E must bind at or very near to the region bound by the anti-51-86 antibodies that OKT4D binds in a region defined by residues 33-65. These data and the direct epitope location data above indicate that the linear order of the epitopes to which the CD4-specific monoclonals bind on the first disulfide loop (see Fig. 1) must be: OKT4C, OKT4A, OKT4D and OKT4E.

The rabbit anti-132-161 antibodies were used to confirm the epitope location of OKT4B and MT151. Pre-binding of either OKT4B or MT151 was able to inhibit the binding of the anti-132-161 antibodies. Although OKT4F maps to the region 132-161, preincubation of this monoclonal with CD4(+) cells had only a slight effect of the binding of the antipeptide antibodies.

Except for OKT4, which has no effect on HIV-1 binding to T cells, all members of the panel of anti-CD4 monoclonal antibodies tested in this study bind to either the first or second of the amino-terminal disulfide loops (see Fig. 1). This result is consistent with the observations of Traunecker et al., (Nature 331:84 (1988)) which indicate that a truncated soluble CD4 protein consisting only of these two domains is sufficient to competitively inhibit HIV-1 from binding to the CD4 protein.

In order to obtain information about the steric interactions of the panel of monoclonals when bound to the CD4 molecule, each monoclonal antibody in our panel was FITC-labeled and tested with all members of the panel to probe for binding competitions. The results of these assays are shown in Figure 4. FITC-OKT4B showed a wide array of competitive binding effects. This may be explained by the fact that this protein is a (pentameric) IgM class antibody and is a very large protein complex. All the other members of the panel are monomeric IgG-type antibodies. Of the antibodies tested, only FITC-OKT4 and FITC-OKT4D were unaffected by the binding of the other monoclonals. In the other cross-competition studies, only two patterns of reciprocal binding interference were observed. OKT4C and OKT4E exhibited mutually competitive binding while OKT4A, which binds near the amino terminal portion of the first disulfide loop, showed reciprocal competitive binding with the CD4-specific monoclonal antibodies which we have mapped to the small disulfide loop, 132-161, i.e., OKT4F and OKT4B.

Results of the cross-competitions between the various monoclonal antibodies are summarized in Figure 1 and are consistent with the idea that the folded structure of the CD4 protein is such that the two disulfide loops must be close to one another. Furthermore, the antibodies which exhibit the strongest effects on the ability of HIV-1 to bind to the CD4 protein, i.e., OKT4A and OKT4F, must have epitopes which are close to one another and lie in a similar plane so that the bound antibodies interfere with one another. It is significant that data from our solid-phase radio-immunoassays show that OKT4A and OKT4C have epitopes which neighbor one another on a linear map (Figure 1), yet the two antibodies display radically different properties (see Table 1) and do not compete with one another in a cross-competition assay, despite the fact that the relative size of each monoclonal antibody is very large compared to the disulfide loops of the CD4 protein. These geometric implications of the epitope location and competitive binding data can be explained by examining the known three dimensional structures of proteins related to the CD4 molecule.

The CD4 protein has been classified as belonging to the immunoglobulin superfamily of proteins on the basis of sequence similarities, both on the nucleic acid and the amino acid levels. See Littman, D. R., Ann. Rev. Immunol. 5:566 (1987). It has been frequently suggested that the amino acid sequence alignments and secondary structure predictions indicate that the folding architecture of the family of immunoglobulin-like proteins is highly conserved (Clark, S. J. et al., Proc. Natl. Acad. Sci. U.S.A. 84:1149 (1987); and Williams, A. F., Immunol. Today 8:298 (1987). For this reason, we examined the three dimensional structures of a series of immunoglobulin regions analogous to residues 1-86 of the CD4 molecule. (The sequence alignments used herein comparing the CD4 protein with the immunoglobulin proteins were derived from published consensus alignments (see Littman, D. R. et al., ibid; Clark, S. J. et al., ibid; and Williams, A. F., ibid). The main chain alpha-carbon backbone of one of those structures, an immunoglobulin light chain variable loop has been elucidated based on the high resolution X-ray diffraction data of Epp et al. (Biochem. 14:4943 (1975)). We have examined all of the three-dimensional immunoglobulin structures which have been deposited in the Brookhaven Database and, although the exact shape and composition of various loops may differ slightly from one protein to the next, the overall folding architecture is conserved and is exemplified by the human IgG structure.

Immunogenic structures are often found to be loops protruding from the surface of a protein domain. Four protruding loops can be identified in all the CD4-like immunoglobulin structures examined and may be designated: 1) Loop 1 corresponding to a region analogous to residues 18-28 of the CD4 protein; 2) Loop 2 analogous to the CD4 residues 30-46; 3) Loop 3 analogous to the CD4 residues 51-62; and 4) Loop 4 analogous to the CD4 residues 63-71. The assignment of these four loops to the epitopes for OKT4C, OKT4A, OKT4D and OKT4E, respectively, is consistent with our epitope location data. Furthermore, cross-competition studies with OKT4C and OKT4E indicated that their respective epitopes must be spatially close and must be oriented such that bound monoclonals interfere with one another. Loop 1 (OKT4C) and Loop 4 (OKT4E) fulfill these criteria. On the other hand, Loop 2 (analogous to residues 30-46), assigned to OKT4A, neighbors Loop 1 (analogous to residues 18-28), assigned to OKT4C, yet the two loops are oriented in opposite directions, consistent with our observation that OKT4A and OKT4C monoclonals do not cross-compete. Finally, Loop 3 (OKT4D) protrudes in such a way that an antibody binding to this loop would not be expected to interfere with the binding of any of the other antibodies. The consistent interpretation of the epitope location and cross-competition data in terms of these structures, together with the sequence alignments and the predicted secondary structure similarities, strengthens the notion that the three-dimensional structure of the first 86 amino acids of the CD4 protein is analogous to the corresponding region of the immunoglobulin structure.

The epitope location and relative orientation data enables us to reach certain conclusions concerning the CD4 interactions with HIV-1. Loop 2 which represents the CD4 epitope assigned to OKT4A, protrudes well away from its surrounding chains such that it looks like an extended finger. An antibody to this "finger" (OKT4A) prevents HIV-1 from binding to the CD4 protein, while an antibody binding to Loop 1 (OKT4C) which is adjacent but points in the opposite direction, has no effect on the binding of HIV-1. The observation that anti-idiotypes of OKT4A bind to the outer envelope of HIV-1 (Kennedy et al., Int. Conf. AIDS III, 160 (1987)) suggests that the binding inhibition observed with OKT4A is not due simply to neighboring steric effects but rather that at least portions of the OKT4A epitope are directly involved in the attachment of HIV-1 to the CD4 protein. By analogy with the picornaviruses (Hogle et al., Science 229: 1358 (1985)) and according to the recent suggestion of Lasky et al. (Cell 50:975 (1987)) in their study of the CD4-binding site on the gp120, it seems likely that the receptor attachment site on the surface of the HIV-1 exists as a valley or invagination. Thus, our data suggests that CD4 residues 30-46 (analogous to Loop 2) form a finger-like projection from the surface of the CD4 protein which serves as a binding site for the HIV-1.

In support of this proposal, the CD4-derived synthetic peptide 18-51 was tested in a HIV-1-induced cell fusion (syncytium formation) assay using HIV-1/WMJ infected cells mixed (1:5) with uninfected SupT1 cells. This peptide was found to specifically inhibit syncytium formation in a concentration-dependent manner, down to concentrations of 200 micrograms/ml. Neither of the peptides -7-18 or 33-65, which were used as controls, were able to inhibit the virus-induced cell fusion at equivalent concentrations. When the peptide 18-51 was washed from the assay two hours after its initiation, syncytium formation proceeded normally, indicating that the peptide was not exerting a toxic effect on the cells and that the observed inhibition was due solely to the presence of the 18-51 peptide.

The invention has been described herein with reference to certain preferred embodiments and examples. However, since obvious variations will appear to those skilled in the art, the invention is not to be considered limited thereto but only by the claims which follow.

**Claims**

1. An HIV binding peptide comprising the amino acid sequence:
Cys-Thr-Ala-Ser-Gln-Lys-Lys-Ser-Ile-Gln-Phe-His-Trp-Lys-Asn-Ser-Asn-Gln-Ile-Lys-Ile-Leu-Gly-Asn-Gln-Gly-Ser-Phe-Leu-Thr-Lys-Gly-Pro-Ser

or any HIV binding analog, fragment, chemical derivative or pharmaceutically acceptable salt thereof.

2. A peptide of claim 1, wherein the amino acid sequence is:

NH₂-Trp-Lys-Asn-Ser-Asn-Gln-Ile-Lys-Ile-Leu-Gly-Asn-Gln-Gly-Ser-Phe-Leu-COOH.

3. A peptide of claim 1, wherein the amino acid sequence is:

NH₂-Cys-Thr-Ala-Ser-Gln-Lys-Lys-Ser-Ile-Gln-Phe-His-Trp-Lys-Asn-Ser-Asn-Gln-Ile-Lys-Ile-Leu-Gly-COOH.

4. An HIV binding peptide comprising the amino acid sequence:

Cys-Arg-Ser-Pro-Arg-Gly-Lys-Asn-Ile-Gln-Gly-Gly-Lys-Thr-Leu-Ser-Val-Ser-Gln-Leu-Glu-Leu-Gln-Asp-Ser-Gly-Thr-Trp-Thr-Cys

or any HIV binding analog, fragment, chemical derivative or pharmaceutically acceptable salt thereof.

5. An antibody specific for a peptide of any preceding claim, wherein said antibody is capable of blocking the binding of HIV-1 to a T4 cell.

6. An anti-idiotype antibody that bears an internal image of a peptide of any of claims 1 to 4 and which is thereby capable of binding to the gp120 envelope protein of HIV-1.

7. A pharmaceutical composition, comprising a peptide of any of claims 1 to 4 and a pharmaceutically acceptable carrier.

8. A vaccine for conferring upon a patient active immunity against HIV, comprising an effective immunizing amount of the antibody of claim 5 and a pharmaceutically acceptable carrier.

9. A diagnostic kit for detecting HIV, comprising the peptide of any of claims 1 to 4.

10. A peptide according to any of claims 1 to 4, for use in therapy.

11. The use of a peptide according to any of claims 1 to 4 in the manufacture of a medicament for treating patients suspected of being infected with HIV.

FIGURE 1

FIGURE 2

CD4-specific peptides

## FIGURE 3

FIGURE 4

FITC—OKT4

FITC—OKT4A

FITC—OKT4C

FITC—OKT4D

FITC—OKT4E

FITC—OKT4F

% OF CELLS STAINED

μg COMPETING ANTIBODY